# EUROPEAN PATENT SPECIFICATION

(11) **EP 3 452 438 B1**
(45) Date of publication and mention of the grant of the patent: **17.11.2021**
(21) Application number: 17792315.8
(22) Date of filing: 15.02.2017
(51) Int. Cl.: C07C 29/151, C10L 3/00, C10L 3/08, C07C 31/04, C25B 1/04

(54) **INTEGRATED TECHNIQUES FOR PRODUCING BIO-METHANOL**
INTEGRIERTES VERFAHREN ZUR HERSTELLUNG VON BIOMETHANOL
TECHNIQUES INTÉGRÉES POUR PRODUIRE DU BIO-MÉTHANOL

(30) Priority: 06.05.2016 US 201662332743 P
(43) Date of publication of application: 13.03.2019
(73) Proprietor: Ultra Clean Ecolene Inc., Kincardine, Ontario N2Z 2Y7 (CA)
(72) Inventor: MACGREGOR, Norman J., Kincardine, Ontario N2Z 2Y7 (CA)
(74) Representative: Cabinet Beau de Loménie
(86) International application number: PCT/CA2017/050192
(87) International publication number: WO 2017/190224

(56) References cited:
- WO-A1-03/051803
- CA-A1- 2 469 653
- CA-A1- 2 703 715
- US-A1- 2008 220 489
- US-A1- 2013 210 937
- US-B2- 8 188 322
- VENKATESWARA RAO ET AL.: 'Biogas generation potential by anaerobic digestion for sustainable energy development in India' RENEWABLE AND SUSTAINABLE ENERGY REVIEWS vol. 14, 2010, pages 2086 - 2094, XP027068805
- KC SURENDRA ET AL.: 'Anaerobic Digestion-Based Biorefmery for Bioenergy and Biobased products' INDUSTRIAL BIOTECHNOLOGY vol. 11, 02 April 2015, XP055438632
- KERI B CANTRELL ET AL.: 'Livestock waste-to-bioenergy generation opportunities' BIORESOURCE TECHNOLOGY vol. 99, 2008, pages 7941 - 7953, XP023182688
- OLUSEGUN A AJAYI ET AL.: 'Methanol Production from Cow Dung' JOURNAL OF ENVIRONMENT AND EARTH SCIENCE vol. 2, no. 7, 2012, pages 9 - 16, XP055438645
- NS SHAMSUL ET AL.: 'An overview on the production of bio-methanol as potential renewable energy' RENEWABLE AND SUSTAINABLE ENERGY REVIEWS vol. 33, 2014, pages 578 - 588, XP055438647

## Description

### TECHNICAL FIELD

The technical field generally relates to the production of methanol, and particularly to integrated processes and systems for producing methanol based biofuel from naturally occurring elements.

### BACKGROUND

Liquid biofuel can be produced from a variety of feedstocks and using various different processing technologies. Energy and reactant requirements for conventional liquid biofuel production techniques can lead to technical and economic challenges as well as elevated fossil fuel emissions.

WO 2003/051803 (Branson) describes a process and apparatus for processing agricultural waste to make alcohol and/or biodiesel. The agricultural wastes are subjected to anaerobic digestion which produces a biogas stream containing methane, which is subsequently reformed to a syngas containing carbon monoxide and hydrogen. The syngas is converted to an alcohol which may be stored, sold, used, or fed directly to a reactor for production of biodiesel.

### SUMMARY

The techniques described herein relate to a route for the production of a liquid biofuel without the engagement of fossil fuels as feedstocks or fossil fuel sourced emissions, and more particularly to integrated processes and systems for producing a liquid hydrocarbon-based sustainable bio-methanol. The techniques enable mitigating fossil fuel derived greenhouse gas emissions from processing and utilization of transportation fuels and commercial or industrial alcohols.

In the present invention according to appended claim 1, a method is provided for producing bio-methanol, comprising:
supplying biomass to an anaerobic digester for producing biogas comprising methane and carbon dioxide;
supplying the biogas and oxygen sourced from water using renewable and/or nuclear-sourced electricity to a partial oxidation unit to produce non fossil fuel-sourced syngas;
supplying the syngas with hydrogen sourced from water using renewable and/or nuclear-sourced electricity to a synthesis unit for producing bio-methanol;
during electricity demand below a base threshold:
   supplying at least a portion of the bio-methanol to storage (e.g., compressed storage) for use as transportation fuel and/or other applications (e.g., commercial/industrial alcohol); and
during electricity demand over a base threshold:
   supplying at least a portion of the bio-methanol to a generator for intermittently producing electricity (e.g., during peak demand);
supplying water to a water electrolysis unit to produce electrolysis oxygen and electrolysis hydrogen;
supplying at least a portion of the electrolysis hydrogen as at least part of the hydrogen used in the synthesis unit; and
supplying at least a portion of the electrolysis oxygen as at least part of the oxygen used in the partial oxidation unit.

In some implementations, the biomass comprises manure, municipal waste, agricultural waste, organic waste, sewerage, purpose grown biomass, and/or cellulose.

In some implementations, the anaerobic digester further produces sulphur and/or fertilizer, and optionally requires supplement heat energy for maximum biogas production.

In some implementations, the process includes heating the anaerobic digester using by-product heat generated by the partial oxidation unit.

In some implementations, the process includes heating the anaerobic digester using by-product heat generated by the water electrolysis unit.

In some implementations, the oxygen supplied to the partial oxidation unit consists of the electrolysis oxygen.

In some implementations, the oxygen supplied to the partial oxidation unit is obtained from an oxygen storage vessel.

In some implementations, the syngas supplied to the synthesis unit consists of the syngas produced by the partial oxidation unit.

In some implementations, the hydrogen supplied to the synthesis unit consists of the electrolysis hydrogen.

In some implementations, the transportation bio-methanol is used as fuel for automobile engines, diesel engines, fuel cells and/or base energy platform for refinery upgrading to aircraft fuel.

In some implementations, the water electrolysis unit further produces deuterium.

In some implementations, at least a portion of the deuterium is supplied to a nuclear reactor facility.

The process of the present invention according to appended claim 1 includes the following:
during electricity demand over an upper value:
   powering the water electrolysis unit using electricity obtained from a generator fuelled with a portion of the stored bio-methanol;
during electricity demand below a lower value:
   powering the water electrolysis unit, and optionally hydrogen and oxygen compressors, using electricity obtained from a source supplied by renewable and/or nuclear energy sources and/or from independent renewable electricity generators.

In some implementations, the base threshold is relatively constant and pre-determined. In some implementations, the upper and lower values are the same. In some implementations, the upper and lower values and the base threshold are the same.

In some implementations, the process includes regulating the base threshold over time to maintain the overall greenhouse gas neutrality of the process.

In some implementations, the process includes controlling electricity input into the water electrolysis unit and controlling the electricity generation from the bio-methanol to maintain the overall greenhouse gas neutrality of the process, and reducing negative impacts of electricity supply/demand characteristics.

The invention according to appended claim 15 provides a system for producing bio-methanol, comprising:
an anaerobic digester unit for producing biogas comprising methane and carbon dioxide;
a partial oxidation unit for receiving the biogas and configured to produce syngas;
a synthesis unit for receiving the syngas and carbon neutral hydrogen, and configured to produce bio-methanol;
a power control assembly configured to
   supply at least a portion of the bio-methanol to a generator for producing electricity, during critical electricity demand over an upper threshold; and supply at least a portion of the bio-methanol to storage for use as transportation fuel, during electricity demand below a lower threshold;
a carbon neutral water electrolysis unit to produce carbon neutral oxygen and hydrogen;
a hydrogen supply and storage assembly configured to supply at least a portion of the electrolysis hydrogen as at least part of the hydrogen used in the synthesis unit; and
an oxygen supply and storage assembly configured to supply at least a portion of the electrolysis oxygen as at least part of the oxygen used in the partial oxidation unit.

### BRIEF DESCRIPTION OF THE DRAWINGS

Figure 1 is a block diagram of an integrated bio-methanol production process with greenhouse gas neutrality.
Figure 2 is a block diagram of a biomass anaerobic digester.
Figure 3 is a block diagram of a water electrolysis unit operation.
Figure 4 is a block diagram of a partial oxidation unit.
Figure 5 is a block diagram of a synthesis unit and tank farm.
Figure 6 is a block diagram of a generator.
Figure 7 is a block diagram of several integrated units and illustrating the electricity source in terms of its supply-demand balance characteristics.
Figure 8 is another block diagram of an integrated bio-methanol production process.
Figure 9 is a block diagram of part of a bio-methanol production process.
Figure 10 is another block diagram of part of a bio-methanol production process.
Figure 11 is another block diagram of part of a bio-methanol production process.
Figure 12 is another block diagram of part of a bio-methanol production process.
Figure 13 is a graph of throughput/production versus electricity source for an example bio-methanol production process.

### DETAILED DESCRIPTION

Various techniques are described herein for bio-methanol production. In some implementations, systems and processes are provided for the production of bio-methanol (which may be referred to here as ECOLENE®). The bio-methanol can be dedicated as a liquid transportation biofuel, as a commercial/industrial alcohol, and/or as a liquid biofuel for generating greenhouse gas neutral electricity particularly during peak electrical demand periods. The bio-methanol can also be dedicated as a liquid storage medium for surplus and low-demand nuclear and/or renewable electricity as well as a novel medium for temporary storage of captured greenhouse gases from decomposed biomass for delayed release back to the atmosphere for balancing via photosynthesis.

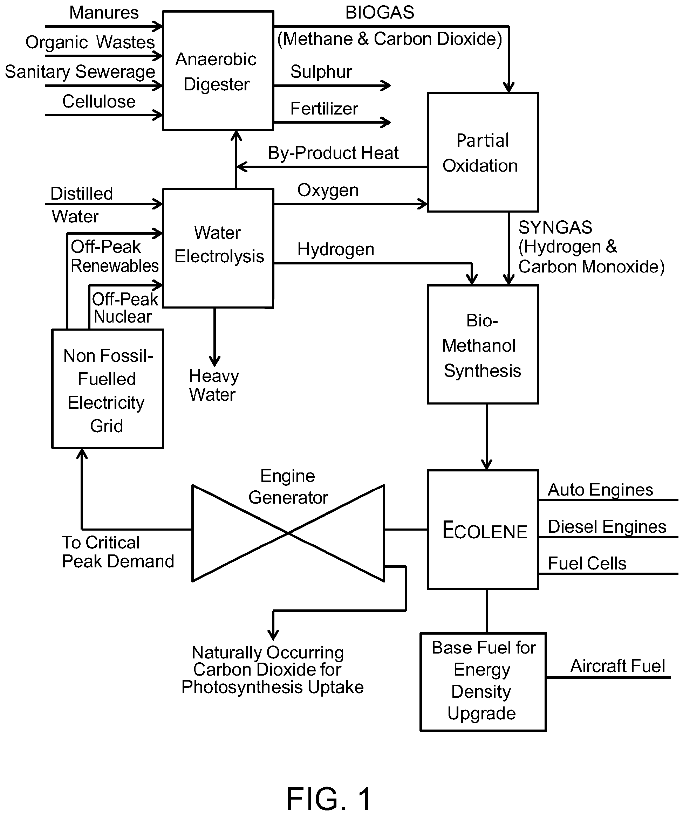

Referring to Figure 1, the system can include integrated units for bio-methanol production and can include an anaerobic digester unit, a partial oxidation unit, a synthesis unit, a storage facility, a water electrolysis unit, and a modulating electricity generator.

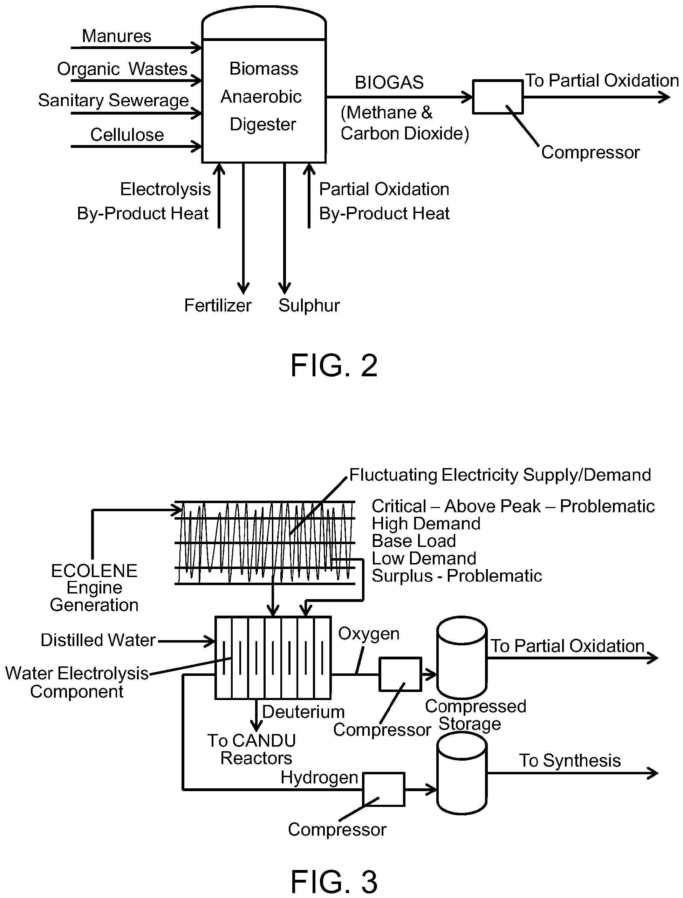

Referring to Figures 1 and 2, in some implementations the anaerobic digester is configured to receive one or more biomass feedstocks, such as manures, organic wastes, sanitary sewerage, cellulose (e.g., pulverized cellulose), and so on. The biomass feedstocks can be sourced locally and can include a combination of different hydrocarbon and carbohydrate sources. The digester can be operated to produce biogas as well as sulphur and fertilizer by-product streams. The sulphur can be harvested incrementally and the composted fertilizer can also be recovered periodically, as byproducts. The fertilizer can be recovered as a coliform-free material and can be processed for sale and/or used in a dedicated biomass production facility (e.g., a greenhouse) that may also use CO₂ that is produced by the process. Both the fertilizer and the CO₂ generated by the process can be stored and then supplied as needed to a biomass production facility (e.g., during certain biomass production cycles). In some cases, the biomass that is produced can then be harvested as part of the feedstock supplied to the anaerobic digester. A biogas storage unit can be provided to receive and store biogas from the digester. A biogas compressor can be provided to operate the digester at or near steady state in order to prevent exhausting and/or flaring of biogas during surplus biogas production periods and other times of the processing. The biogas storage can be monitored and controlled to retrieve and supply controlled amounts of the biogas to the partial oxidation unit, for example. Such control can also incorporate input from other process units. The biogas production can be monitored and controlled to obtain a composition within a pre-determined range, particularly with respect to the stoichiometric balance of methane and carbon dioxide, for example to maximize production and utilization.

In some implementations, biogas can be burned directly in the generator, for example in periods of biogas overproduction and/or during outages of partial oxidation and/or synthesis reactors to avoid emissions. The generator unit can include combustion generator devices that are adapted to receive biogas and/or bio-methanol streams as fuel (alternately and/or simultaneously), and/or the generator unit can include multiple generator devices each dedicated to a given fuel (e.g., a biogas-receiving generator, a bio-methanol-receiving generator, etc.).

Referring to Figures 1 and 3, in some implementations the water electrolysis unit is configured to receive distilled water and electricity from non-fossil fuel sources. The water can be obtained from a water distillation unit or another type of water purification unit that may be located on site or proximate to the water electrolysis unit, for example. Energy required for water distillation can be obtained in whole or in part from renewable sources, such as biomass or bio-methanol combustion. The water electrolysis unit can be fully variable, fully interruptible and outfitted with compressors and storage vessels to ensure a constant regulated supply of output (oxygen and hydrogen) are available during interruption and/or high electricity demand periods. By-product heat from the water electrolysis unit can be captured and delivered to the digester and/or to pretreatment units for pre-treating the biomass prior to entering the digester. The by-product heat recovery can facilitate temperature control of the digester for optimizing microbial production when appropriate. The by-product heat can be supplied to cooling fans or towers when the heat is not required elsewhere in the process. In addition, the water electrolysis unit can include deuterium harvesting capability, for recovering deuterium (heavy water) for use as a heat transfer medium and/or in medical applications. The water electrolysis unit can thus be configured and operated to promote production of deuterium-rich liquid. For example, the water electrolysis unit can include a cascade of electrolysis chambers for concentrating the deuterium in each subsequent chamber until pure deuterium is produced, or there may be a separate deuterium harvester/separator that is coupled to the water electrolysis unit to receive deuterium-enriched liquid that can be further separated into a substantially pure deuterium via chemical exchange and/or distillation methods. The electrolysis-derived heavy water can be used in a nuclear reactor heat transfer system (e.g., part of a CANDU™ facility).

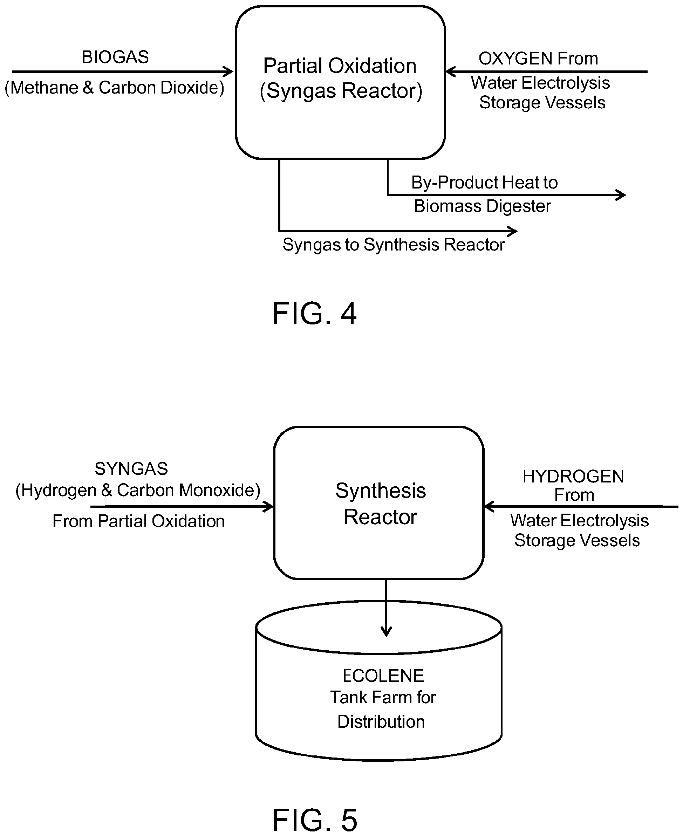

Referring to Figures 1 and 4, in some implementations the partial oxidation unit is fluidly connected with the biogas storage facility and/or the digester, to receive biogas to be burned using compressed oxygen sourced from the water electrolysis unit to produce syngas comprising or substantially consisting of hydrogen and carbon monoxide.

Referring to Figures 1 and 5, in some implementations the syngas together with compressed hydrogen from water electrolysis are supplied to a synthesis unit configured to produce non fossil fuel-based bio-methanol, which may be referred to herein as ECOLENE®.

Still referring to Figures 1, 5 and 6, the bio-methanol can be supplied to a storage facility, e.g., tank farm, which can be monitored and controlled in various ways that will be described herein. The bio-methanol storage facility can be configured for distribution as well as periodic supply to a generator for electricity generation. In some implementations, the bio-methanol storage facility is configured with sufficient tank storage inventory or capacity to enable periodic electricity generation, for example during critical peak demand. The tank storage capacity can therefore be co-ordinated with electrolysis electricity demand and peak non fossil fuelled electricity demand. The storage facility can also include piping, monitoring instrumentation, pumps and control units to manage the storage and the supply of the bio-methanol.

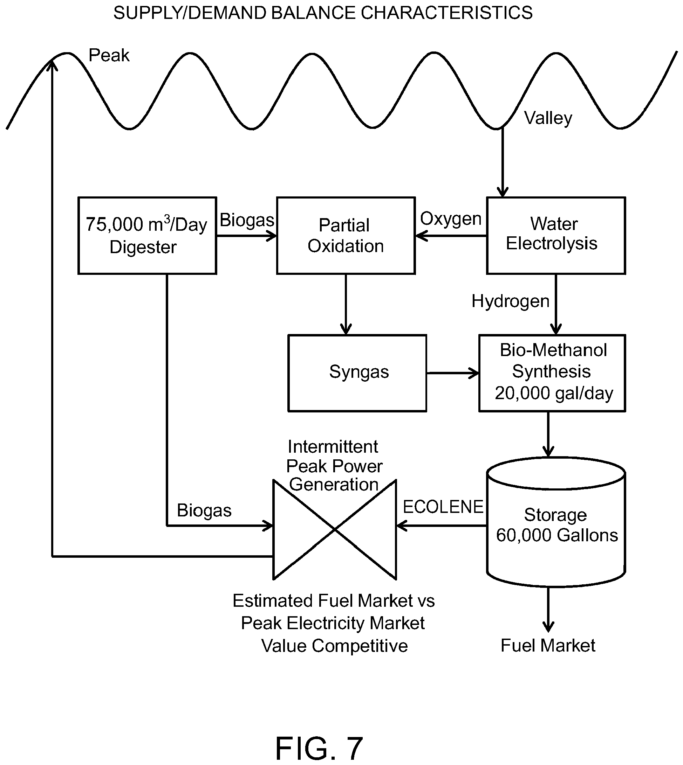

In some implementations, the capacity to intermittently utilize surplus and/or low demand electricity in variable amounts to produce non fossil-sourced hydrocarbons with the capacity to intermittently generate critical and high demand electricity in variable amounts can facilitate the increasing need to balance electricity supply with electricity demand. The capacity to produce bio-methanol during low electricity demand and use the bio-methanol to generate electricity during high electricity demand will help reduce demand charges and improve the quality of electricity. In some scenarios, time-of-day pricing by electricity system operators can be used to determine the value for using surplus electricity capacity for purchasing low demand electricity and a charge for demand. The capacity to generate electricity using bio-methanol ECOLENE® and/or biogas can be determined by the steady state capacity of the biogas using ECOLENE® as a back-up biofuel. For example, a 76,000 litres/day (20,000 US gal/day) "regional" bio-methanol plant may use 75,000 m³ biogas/day, which is generally reflected in Figure 7.

Time-of-use pricing of electricity can vary depending on various factors and locations. For example, in some jurisdictions, off-peak electricity rates can apply from approximately 8:00PM-7:00AM and can have a cost that is about 65-75% of the mid-peak rate and about 30-55% of the on-peak rate.

In some implementations, the capacities of the different units can be coordinated with factors based on electricity demand cycles, estimated fuel market, and the like. In some scenarios, the digester is sized and operated to produce between 25,000 m³/day and 200,000 m³/day biogas, or between 50,000 m³/day and 100,000 m³/day biogas; the bio-methanol synthesis unit is sized and operated to produce between 19,000 litres/day (5,000 gal/day) and 380,000 litres/day (100,000 gal/day) of bio-methanol, or between 57,000 litres/day (15,000 gal/day) and 95,000 litres/day (25,000 gal/day); and the biomethanol storage facility has a capacity of between 57,000 litres (15,000 gallons) and 380,000 litres (100,000 gallons), or between 151,000 litres (40,000 gallons) and 303,000 litres (80,000) gallons of the biofuel. Subject to biomass availability, much larger biomethanol plants can be implemented in the proximity of large nuclear and/or renewable electricity generating sites.

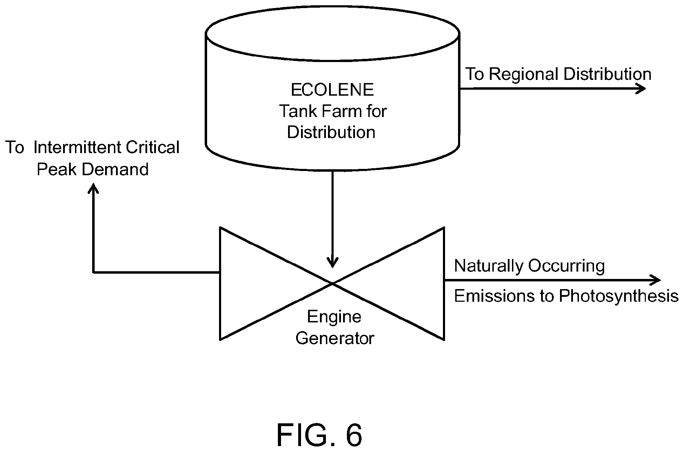

Referring to Figure 6, a generator can be provided to receive bio-methanol from the storage facility and provide electricity to the water electrolysis unit. The generator may be specially designed and dedicated for the combustion of bio-methanol to produce electricity without emitting fossil fuel sourced greenhouse gases. The generator can be configured to receive different fuels, which may be liquid non fossil-sourced fuels only or a combination of liquid non fossil-sourced fuels including biogas. The combustion of the bio-methanol and/or biogas would be substantially free of fossil sourced greenhouse gas emissions that would be associated with the combustion of fossil fuels, for example. By-product heat from the generator can also be used in the process, e.g., for optimizing the microbial production in the digester.

An integration assembly can be provided to integrate different units of the system. For example, the integration assembly can include the generator, inlet biomethanol fuel piping, electrical supply lines for supplying bio-methanol generated electricity to the water electrolysis unit, a control unit coupled to the piping and/or valves for controlling the periodic operation of the generator, which may be done according to input variables that include electricity demand levels to determine the timing of peak demand, as well as various detection and monitoring devices such as temperature sensors, pressure sensors and/or flow rate meters and/or actuators. The integration assembly may include an automation apparatus, such as a computer, configured to control the integration automatically in response to the input variables to ensure pressure/temperature and processing duration for the conversion process (e.g., space, gas, velocity).

Various techniques described herein can be used in the context of a carbon capture, carbon storage, carbon trade, carbon credit, and carbon tax systems.

Production of ECOLENE® can enable a liquid hydrocarbon to be commercially synthesized by controlled digestion of waste biomass as feedstock to capture and utilize methane and carbon dioxide to produce a biofuel rather than enter the atmosphere directly as greenhouse gases. By utilizing only renewable- and/or nuclear-sourced electricity, to decompose water to produce the essential elements of hydrogen and oxygen, unlike other methanol synthesis processes which use fossil fuel-sourced input streams, ECOLENE® production enables its emissions of carbon dioxide to remain more in atmospheric balance through photosynthesis.

In some implementations, the system can be a regional hub that is located to serve a remote solar farm, a remote hydraulic generation facility, a remote wind farm and/or an ocean energy facility where conventional grids or related infrastructure are inadequate or do not exist. Bio-methanol can thus be a particularly advantageous source of electricity storage and/or a liquid carrier/transporter of electron energy.

In some implementations, the bio-methanol can also be used as a liquid fuel for various conventional and/or hybrid transportation power trains, as well as other methods. Thus, using biomass, water and variable volumes of renewable and/or nuclear sourced electricity during low electricity system demand, as described herein, can enable biomethanol to be used to power internal combustion engines for conventional power trains, on-board generators for hybrid and/or all electric power trains, carry hydrogen for fuel cell powered electric drives and/or generate electricity during high electricity demand, qualifying such bio-methanol to be a liquid electricity storage medium "battery". Biomethanol production, storage inventory and distribution can be managed to facilitate a plurality of end-uses that can be coordinated with advantageous time periods (e.g., electricity demand cycles), locations (e.g., regional, infrastructure-deficient, etc.), as well as various cost/economic factors.

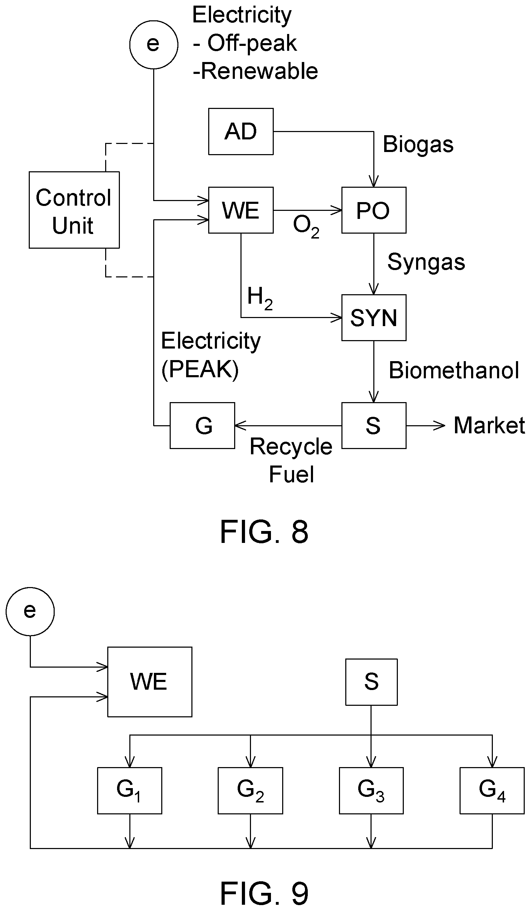

Referring to Figure 8, the overall bio-methanol fuel production process is illustrated where a control unit is coupled to both the electrical output of the generator (G) and an electrical line from an external electricity source (e), which may include electricity from an electricity grid dominated with renewable sources to ensure the electricity flow is carbon neutral. The control unit can be configured to receive information regarding the bio-methanol production process as well as the external electricity source(s), including cost information for external electricity as well as for inputs (e.g., biomass feedstocks) and outputs (e.g., bio-methanol) of the production system. The control unit can be configured to balance the electricity sources (i.e., internal and external) to minimize cost or to reduce cost while prioritizing more sustainable electricity sources.

Referring to Figure 9, a water electrolysis unit (WE) can receive electricity from both external sources (e) and internal sources (G₁ to Gₙ). In some scenarios, it may be advantageous to provide multiple generators (G₁ to Gₙ) which can be operated individually or together depending on the electricity demand from the water electrolysis unit (WE). For example, during high throughput/production periods and peak demand, multiple or all of the generators can be operated to produce electricity; while during lower throughput/production periods and/or off-peak, only some or none of the generators can be operated to produce electricity. Multiple smaller generators, all of which can be coupled to a central control unit, can thus be used in a modular fashion to tailor the electricity generation in a flexible manner than can adapt to both external electricity cost and availability and the production mode (e.g., high production, start-up, turndown, upset, etc.) of the bio-methanol production process.

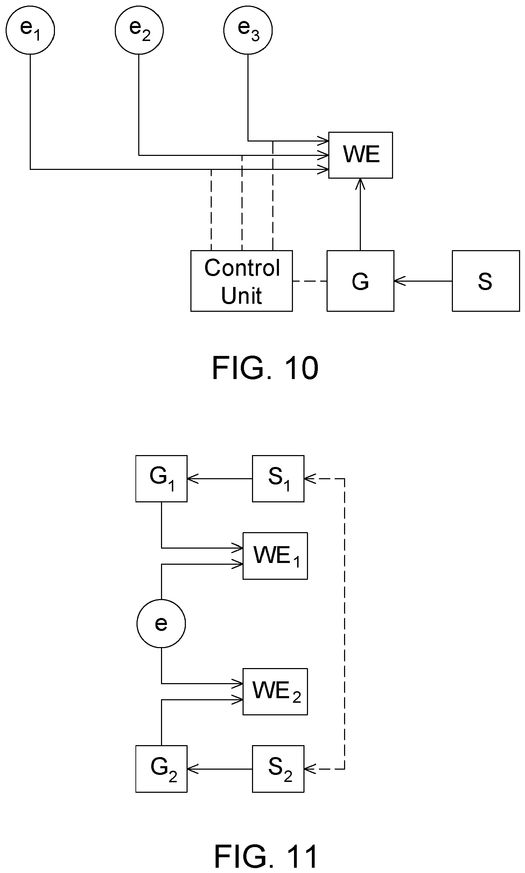

Referring to Figure 10, the water electrolysis unit (WE) can be coupled to multiple external electricity sources (e₁ to e₃), each of which can originate from a different electricity generation method. For example, a first external electricity source (e₁) may be wind-generated, a second external electricity source (e₂) may be hydro-generated, a third external electricity source (e₃) may be nuclear-generated, while other external electricity sources may come from various other renewable sources, some of which have been mentioned above. By coupling the bio-methanol production process to multiple external electricity sources, access to renewable electricity can be more robust particularly when some of the output from the renewable sources is inconsistent or difficult to predict in terms of availability and/or cost. For example, certain renewable energy sources are weather dependent (e.g., wind) and thus by providing multiple external sources, the process can operate more efficiently. In addition, the control unit can be configured to select and balance the electricity sources that are used for the water electrolysis unit based on fluctuations in each external electricity source.

Referring to Figure 11, multiple water electrolysis units can be provided and in some cases can employ one or more common external electricity source (e). The multiple water electrolysis units can be part of the same overall bio-methanol production process or they can be part of two distinct and potentially remote processes, e.g., provided in two different regional locations. Each water electrolysis unit (WE₁ and WE₂) can be coupled to its own generator (G₁ and G₂ respectively), which can in turn be coupled to two different storage facilities (S₁ and S₂ respectively) or to a single central storage facility. This general configuration can be particularly advantageous for implementing multiple bio-methanol production plants in a plurality of remote locations that are nevertheless serviced by a common electrical grid and/or by common external electrical sources. In addition, a bank of generators can include a primary generator as well as backup generators, which can come online quickly and periodically to facilitate avoiding spikes in peak demand. Multiple generators can thus be particularly advantageous when there are sudden, large and/or unpredictable spikes in peak demand, by facilitating rapid adjustment.

In some implementations, the primary generator (G₁) can be designed and provided to be able to respond to normal electricity requirements during peak demand periods and typical operation of the bio-methanol production plant, while a secondary or backup generator (G₂) is a smaller unit designed for more occasional operation during sudden peaks, emergency demand periods, and/or when bio-methanol price is lower than external electricity cost. In some implementations, one or more generators can be designed to utilize the bio-methanol as the dedicated fuel, while one or more additional generators are provided for use with other fuel sources (e.g., biogas) or as fuel-neutral units that can receive methanol, biogas and/or other fuel sources for electricity generation.

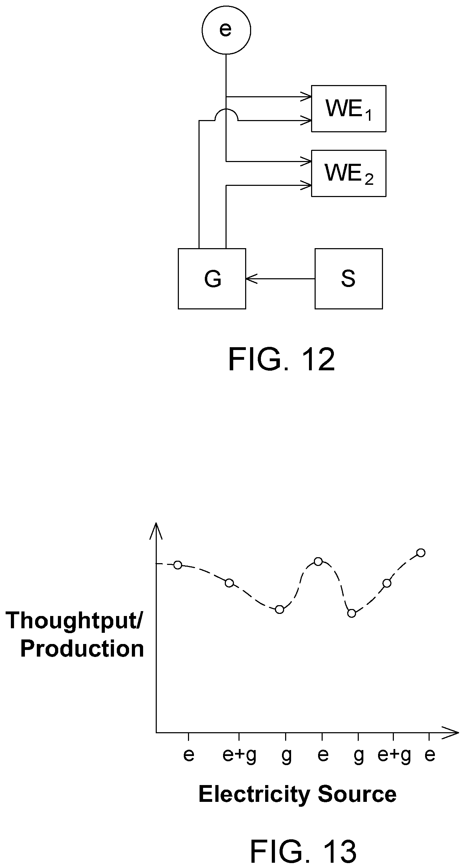

Referring to Figure 12, the bio-methanol production process can include multiple water electrolysis units (WE₁ and WE₂) that are part of the same production plant and are operated in accordance with electricity sourcing strategy and the bio-methanol production mode. For example, during low throughput/production (e.g. during start-up or turndown modes, maintenance, or feedstock modification) a single water electrolysis unit may be used and it may be supplied with electricity based on the above-described methods by using off-peak electricity from the external source (e) and bio-methanol generated electricity during peak periods. As the production process ramps up, the second water electrolysis unit can come online and can be supplied by both external and internal sources of electricity, as described above. A bank of multiple water electrolysis units can provide additional flexibility for bio-methanol production processes, particularly when the plants have variable throughputs and production.

In addition, the production rate of the process can also be controlled based on electricity availability and cost. For example, during peak demand, the production rate can be decreased in conjunction with using bio-methanol to generate electricity for operating the water electrolysis unit(s). This can be particularly advantageous in the case that the bio-methanol market price is high and/or when the biomass feedstock cost is high, thereby reducing the consumption of bio-methanol for generating electricity while keeping the process operational during peak demand periods. Alternatively, when biomethanol price and feedstock cost are low, the production rate can be maintained at substantially the same levels as during off-peak operations.

Turning to Figure 13, an example of modulating throughput and production rate of the process based on the different electricity inputs (e) and/or (G) is illustrated. One can also integrate the cost of biomass feedstocks and the price of the bio-methanol into the control strategy which can be implemented in automated fashion by a control unit that is coupled to the various units of the process.

Advantageously, off-peak external electricity consists of electricity from non-fossil fuel sources. Various examples of non-fossil fuel sources of electricity are provided further above. Further examples are (i) when nuclear reactors are modulated or when primary nuclear sourced steam is being quenched, (ii) when wind energy generation is being strategically curtailed, (iii) when hydro-energy is being spilled as part of a supply management strategy. A number of variable electricity sources can be used.

In addition, since water electrolysis units can incrementally and quickly modulate demand, utilizing water electrolysis units in the context of the techniques described herein facilitates critical load manipulation. Electrolysis interruption is ideally avoided and thus leveraging the bio-methanol for generating electricity dedicated for maintaining electrolysis operation facilitates efficient operation of the process.

In some implementations, the generator (G) is a dedicated bio-methanol combustion unit that is designed and operated for use with 100% methanol as fuel. Alternatively, the generator can be used for various different fuel types, including methanol. In some implementations, the combustion gas generated by the generator(s) is recuperated and reused either within the bio-methanol production process or in other processes. For instance, in some scenarios, the CO₂ in the combustion gas can be separated and reused in the process, in another system (e.g., greenhouses for photosynthesis and production of biomass), and/or in a capture/sequestration system. The CO₂ in the combustion gas can be prepared and supplied directly to a CO₂-utilization facility or can be captured from the combustion gas and stored as substantially pure CO₂ for use. Heat generated by the generator can also be used in a biomass generation facility, such as a greenhouse, or other external or internal units. In some scenarios, at least one of the generators can be portable to facilitate relocation as need be, e.g., between two remote process locations.

Units and components of the systems described herein can also be used and configured in various ways. For example, certain unit operations can be provided as a serial or parallel bank of units. Another example is that processes described herein can be adapted for production of liquid biofuel other than bio-methanol by periodically using liquid biofuel as a source of electricity for one or more units during peak demand periods, particularly when such electricity is supplied to a water electrolysis unit or another unit having similar electricity requirements. In addition, multiple generators can be provided in parallel in order to process different amounts of bio-methanol to produce electricity for the water electrolysis unit depending on the electricity demand, the electrolysis electricity demand and/or the inventory of bio-methanol.

## Claims

1. A method for producing bio-methanol, comprising:
supplying biomass to an anaerobic digester for producing biogas comprising methane and carbon dioxide;
supplying the biogas and oxygen sourced from water using renewable and/or nuclear-sourced electricity to a partial oxidation unit to produce non fossil fuel-sourced syngas;
supplying the syngas with hydrogen sourced from water using renewable and/or nuclear-sourced electricity to a synthesis unit for producing bio-methanol;
during electricity demand below a base threshold:
supplying at least a portion of the bio-methanol to storage; and
during electricity demand over a base threshold:
supplying at least a portion of the bio-methanol to a generator for intermittently producing bio-sourced electricity;
supplying distilled water to a water electrolysis unit to produce electrolysis oxygen and electrolysis hydrogen;
supplying at least a portion of the electrolysis hydrogen as at least part of the hydrogen used in the synthesis unit; and
supplying at least a portion of the electrolysis oxygen as at least part of the oxygen used in the partial oxidation unit; and
wherein:
during electricity demand over an upper value:
powering the water electrolysis unit using electricity obtained from a generator fuelled with a portion of the stored bio-methanol; and
during electricity demand below a lower value:
powering the water electrolysis unit, and optionally hydrogen and oxygen compressors, using electricity obtained from a source supplied by renewable and/or nuclear energy sources and/or from independent renewable electricity generators.

2. The method of claim 1, wherein the biomass comprises manure, organic waste, sewerage and/or cellulose.

3. The method of claim 1 or 2, wherein the anaerobic digester further produces sulphur and/or fertilizer.

4. The method of any one of claims 1 to 3, further comprising heating the anaerobic digester using by-product heat generated by the partial oxidation unit or using by-product heat generated by the water electrolysis unit.

5. The method of any one of claims 1 to 4, wherein the oxygen supplied to the partial oxidation unit consists of the electrolysis oxygen.

6. The method of any one of claims 1 to 5, wherein the syngas supplied to the synthesis unit consists of the syngas produced by the partial oxidation unit.

7. The method of any one of claims 1 to 6, wherein the hydrogen supplied to the synthesis unit consists of the electrolysis hydrogen.

8. The method of any one of claims 1 to 7, wherein the water electrolysis unit further produces deuterium.

9. The method of claim 8, wherein at least a portion of the deuterium is supplied to a nuclear reactor facility.

10. The method of any one of claims 1 to 9, wherein the base threshold is relatively constant and pre-determined.

11. The method of any one of claims 1 to 9, wherein the upper and lower values are the same.

12. The method of any one of claims 1 to 9, further comprising regulating the base threshold over time to maintain overall greenhouse gas neutrality of the process.

13. The method of any one of claims 1 to 12, further comprising:
controlling electricity input into the water electrolysis unit and controlling the electricity generation from the bio-methanol to maintain overall greenhouse gas neutrality of the process, and reducing negative impacts of electricity demand characteristics.

14. The method of any one of claims 1 to 13, wherein the upper and lower values and the base threshold are the same.

15. A system for producing bio-methanol, comprising:
an anaerobic digester unit for producing biogas comprising methane and carbon dioxide;
a partial oxidation unit for receiving the biogas and configured to produce syngas;
a synthesis unit for receiving the syngas and carbon neutral hydrogen, and configured to produce bio-methanol;
a power control assembly configured to
supply at least a portion of the bio-methanol to a generator for producing electricity, during critical electricity demand over an upper threshold; and supply at least a portion of the bio-methanol to storage for use as transportation fuel or as a commercial or industrial alcohol, during electricity demand below a lower threshold;
a carbon neutral water electrolysis unit to produce carbon neutral oxygen and hydrogen;
a hydrogen supply and storage assembly configured to supply at least a portion of the electrolysis hydrogen as at least part of the hydrogen used in the synthesis unit; and
an oxygen supply and storage assembly configured to supply at least a portion of the electrolysis oxygen as at least part of the oxygen used in the partial oxidation unit.

## Patentansprüche

1. Verfahren zur Herstellung von Biomethanol, umfassend:
Zuführen von Biomasse zu einem anaeroben Fermenter zur Herstellung von Biogas, das Methan und Kohlendioxid umfasst,
Zuführen des Biogases und von aus Wasser unter Verwendung von Elektrizität aus erneuerbarer und/oder nuklearer Quelle erzeugtem Sauerstoff zu einer Teiloxidationseinheit, um Synthesegas, das nicht auf fossilem Brennstoff beruht, herzustellen,
Zuführen des Synthesegases mit von aus Wasser unter Verwendung von Elektrizität aus erneuerbarer und/oder nuklearer Quelle erzeugtem Wasserstoff zu einer Syntheseeinheit, um Biomethanol herzustellen,
während einer Elektrizitätsnachfrage unterhalb eines Basisschwellenwerts:
Zuführen mindestens eines Anteils des Biomethanols zu einem Speicher, und
während einer Elektrizitätsnachfrage oberhalb eines Basisschwellenwerts:
Zuführen mindestens eines Anteils des Biomethanols zu einem Generator, um intermittierend Elektrizität aus biologischer Quelle herzustellen,
Zuführen von destilliertem Wasser zu einer Wasserelektrolyseeinheit, um Elektrolysesauerstoff und Elektrolysewasserstoff herzustellen,
Zuführen mindestens eines Anteils des Elektrolysewasserstoffs als mindestens einen Teil des in der Syntheseeinheit verwendeten Wasserstoffs, und
Zuführen mindestens eines Anteils des Elektrolysesauerstoffs als mindestens einen Teil des in der Teiloxidationseinheit verwendeten Sauerstoffs, und
wobei:
während einer Elektrizitätsnachfrage oberhalb eines oberen Werts:
die Wasserelektrolyseeinheit unter Verwendung von Elektrizität angetrieben wird, die von einem mit einem Anteil des gespeicherten Biomethanols betriebenen Generator erhalten wird, und
während einer Elektrizitätsnachfrage unter einem unteren Wert:
die Wasserelektrolyseeinheit und optional Wasserstoff- und Sauerstoffkompressoren unter Verwendung von Elektrizität angetrieben werden, die von einer Quelle erhalten wird, die durch erneuerbare und/oder nukleare Energiequellen und/oder von unabhängigen erneuerbaren Elektrizitätsgeneratoren versorgt wird.

2. Verfahren nach Anspruch 1, wobei die Biomasse Gülle, organische Abfälle, Abwasser und/oder Cellulose umfasst.

3. Verfahren nach Anspruch 1 oder 2, wobei der anaerobe Fermenter ferner Schwefel und/oder Düngemittel erzeugt.

4. Verfahren nach einem der Ansprüche 1 bis 3, das ferner das Erhitzen des anaeroben Fermenters unter Verwendung von Nebenproduktwärme, die von der Teiloxidationseinheit erzeugt wird, oder unter Verwendung von Nebenproduktwärme, die von der Wasserelektrolyseeinheit erzeugt wird, umfasst.

5. Verfahren nach einem der Ansprüche 1 bis 4, wobei der der Teiloxidationseinheit zugeführte Sauerstoff aus dem Elektrolysesauerstoff besteht.

6. Verfahren nach einem der Ansprüche 1 bis 5, wobei das Synthesegas, das der Syntheseeinheit zugeführt wird, aus dem von der Teiloxidationseinheit hergestellten Synthesegas besteht.

7. Verfahren nach einem der Ansprüche 1 bis 6, wobei der der Syntheseeinheit zugeführte Wasserstoff aus dem Elektrolysewasserstoff besteht.

8. Verfahren nach einem der Ansprüche 1 bis 7, wobei die Wasserelektrolyseeinheit ferner Deuterium erzeugt.

9. Verfahren nach Anspruch 8, wobei mindestens ein Anteil des Deuteriums einer Nuklearreaktoranlage zugeführt wird.

10. Verfahren nach einem der Ansprüche 1 bis 9, wobei der Basisschwellenwert relativ konstant und vorbestimmt ist.

11. Verfahren nach einem der Ansprüche 1 bis 9, wobei der obere und der untere Wert gleich sind.

12. Verfahren nach einem der Ansprüche 1 bis 9, das ferner das Regeln des Basisschwellenwerts mit der Zeit umfasst, um die Gesamttreibhausgasneutralität des Prozesses zu bewahren.

13. Verfahren nach einem der Ansprüche 1 bis 12, ferner umfassend:
Steuern der Elektrizitätseinspeisung in die Wasserelektrolyseeinheit und Steuern der Elektrizitätserzeugung von dem Biomethanol, um die Gesamttreibhausgasneutralität des Prozesses zu bewahren und negative Auswirkungen von Elektrizitätsnachfragekennlinien zu vermindern.

14. Verfahren nach einem der Ansprüche 1 bis 13, wobei der obere und der untere Wert und der Basisschwellenwert gleich sind.

15. System zur Herstellung von Biomethanol, umfassend:
eine anaerobe Fermentereinheit zur Herstellung von Biogas, das Methan und Kohlendioxid umfasst,
eine Teiloxidationseinheit zum Empfangen des Biogases und die dazu ausgestaltet ist, Synthesegas herzustellen,
eine Syntheseeinheit zum Empfangen des Synthesegases und kohlenstoffneutralen Wasserstoffs und die dazu ausgestaltet ist, Biomethanol herzustellen,
eine Leistungssteuerungsanordnung, die ausgestaltet ist zum:
Zuführen mindestens eines Anteils des Biomethanols zu einem Generator zur Herstellung von Elektrizität während einer kritischen Elektrizitätsnachfrage über einem oberen Schwellenwert, und
Zuführen mindestens eines Anteils des Biomethanols zu einem Speicher zur Verwendung als Transportkraftstoff oder als technischer oder Industriealkohol während einer Elektrizitätsnachfrage unter einem unteren Schwellenwert,
eine kohlenstoffneutrale Wasserelektrolyseeinheit, um kohlenstoffneutralen Sauerstoff und Wasserstoff herzustellen,
eine Wasserstoffzufuhr- und Speicheranordnung, die dazu ausgestaltet ist, mindestens einen Anteil des Elektrolysewasserstoffs als mindestens einen Teil des in der Syntheseeinheit verwendeten Wasserstoffs zuzuführen, und
eine Sauerstoffzufuhr- und Speicheranordnung, die dazu ausgestaltet ist, mindestens einen Anteil des Elektrolysesauerstoffs als mindestens einen Teil des in der Teiloxidationseinheit verwendeten Sauerstoffs zuzuführen.

## Revendications

1. Procédé pour la production de bio-méthanol, comprenant :
la fourniture de biomasse dans un digesteur anaérobie pour produire du biogaz comprenant du méthane et dioxyde de carbone ;
la fourniture du biogaz et d'oxygène provenant d'eau en utilisant de l'électricité renouvelable et/ou de source nucléaire dans une unité d'oxydation partielle pour produire du gaz de synthèse de source combustible non fossile ;
la fourniture du gaz de synthèse avec de l'hydrogène provenant d'eau en utilisant de l'électricité renouvelable et/ou de source nucléaire dans une unité de synthèse pour produire du bio-méthanol ;
pendant la demande d'électricité en dessous d'un seuil de base :
la fourniture d'au moins une portion du bio-méthanol dans un stockage ; et
pendant une demande d'électricité au-dessus d'un seuil de base :
la fourniture d'au moins une portion du bio-méthanol dans un générateur pour produire de façon intermittente de l'électricité de source bio ;
la fourniture d'eau distillée dans une unité d'électrolyse d'eau pour produire de l'oxygène d'électrolyse et de l'hydrogène d'électrolyse ;
la fourniture d'au moins une portion de l'hydrogène d'électrolyse comme au moins une partie de l'hydrogène utilisé dans l'unité de synthèse ; et
la fourniture d'au moins une portion de l'oxygène d'électrolyse comme au moins une partie de l'oxygène utilisé dans l'unité d'oxydation partielle ; et
dans lequel :
pendant la demande d'électricité au-dessus d'une valeur supérieure :
la mise en marche de l'unité d'électrolyse d'eau en utilisant de l'électricité obtenue à partir d'un générateur alimenté en combustible avec une portion du bio-méthanol stocké ; et
pendant la demande d'électricité en dessous d'une valeur inférieure :
la mise en marche de l'unité d'électrolyse d'eau, et éventuellement de compresseurs d'hydrogène et d'oxygène, en utilisant de l'électricité obtenue à partir d'une source alimentée par des sources d'énergie renouvelable et/ou nucléaire et/ou à partir de générateurs d'électricité renouvelable indépendants.

2. Procédé selon la revendication 1, dans lequel la biomasse comprend du fumier, des déchets organiques, des eaux usées et/ou de la cellulose.

3. Procédé selon la revendication 1 ou 2, dans lequel le digesteur anaérobie produit de plus du soufre et/ou de l'engrais.

4. Procédé selon l'une quelconque des revendications 1 à 3, comprenant de plus le chauffage du digesteur anaérobie en utilisant de la chaleur de produit secondaire générée par l'unité d'oxydation partielle ou en utilisant de la chaleur de produit secondaire générée par l'unité d'électrolyse d'eau.

5. Procédé selon l'une quelconque des revendications 1 à 4, dans lequel l'oxygène fourni dans l'unité d'oxydation partielle consiste en l'oxygène d'électrolyse.

6. Procédé selon l'une quelconque des revendications 1 à 5, dans lequel le gaz de synthèse fourni dans l'unité de synthèse consiste en le gaz de synthèse produit par l'unité d'oxydation partielle.

7. Procédé selon l'une quelconque des revendications 1 à 6, dans lequel l'hydrogène fourni dans l'unité de synthèse consiste en l'hydrogène d'électrolyse.

8. Procédé selon l'une quelconque des revendications 1 à 7, dans lequel l'unité d'électrolyse d'eau produit de plus du deutérium.

9. Procédé selon la revendication 8, dans lequel au moins une portion du deutérium est fournie dans une installation de réacteur nucléaire.

10. Procédé selon l'une quelconque des revendications 1 à 9, dans lequel le seuil de base est relativement constant et pré-déterminé.

11. Procédé selon l'une quelconque des revendications 1 à 9, dans lequel les valeurs supérieure et inférieure sont identiques.

12. Procédé selon l'une quelconque des revendications 1 à 9, comprenant de plus la régulation du seuil de base au cours du temps pour maintenir une neutralité globale des gaz à effet de serre du procédé.

13. Procédé selon l'une quelconque des revendications 1 à 12, comprenant de plus :
le contrôle d'alimentation en électricité dans l'unité d'électrolyse d'eau et le contrôle de la production d'électricité à partir du bio-méthanol pour maintenir une neutralité globale des gaz à effet de serre du procédé, et la réduction d'impacts négatifs des caractéristiques de demande d'électricité.

14. Procédé selon l'une quelconque des revendications 1 à 13, dans lequel les valeurs supérieure et inférieure et le seuil de base sont identiques.

15. Système pour la production de bio-méthanol, comprenant :
une unité de digesteur anaérobie pour produire du biogaz comprenant du méthane et du dioxyde de carbone ;
une unité d'oxydation partielle pour recevoir le biogaz et configurée pour produire du gaz de synthèse ;
une unité de synthèse pour recevoir le gaz de synthèse et de l'hydrogène neutre en carbone, et configurée pour produire du bio-méthanol ;
un assemblage de contrôle de puissance configuré pour
fournir au moins une portion du bio-méthanol dans un générateur pour produire de l'électricité, pendant une demande d'électricité critique au-dessus d'un seuil supérieur ; et
fournir au moins une portion du bio-méthanol dans un stockage pour une utilisation comme un combustible de transport ou comme un alcool commercial ou industriel, pendant une demande d'électricité en dessous d'un seuil inférieur ;
une unité d'électrolyse d'eau neutre en carbone pour produire de l'oxygène et de l'hydrogène neutre en carbone;
un ensemble de fourniture et stockage d'hydrogène configuré pour fournir au moins une portion de l'hydrogène d'électrolyse comme au moins une partie de l'hydrogène utilisé dans l'unité de synthèse ; et
un ensemble de fourniture et stockage d'oxygène configuré pour fournir au moins une portion de l'oxygène d'électrolyse comme au moins une partie de l'oxygène utilisé dans l'unité d'oxydation partielle.
